# EUROPEAN PATENT SPECIFICATION

(11) **EP 1 873 232 B1**
(45) Date of publication and mention of the grant of the patent: **26.02.2020**
(21) Application number: 06124425.7
(22) Date of filing: 20.11.2006
(51) Int. Cl.: C12M 3/00

(54) **Microinjection apparatus and automatic focal point adjustment method**
Mikroinjektionsvorrichtung und automatisches Brennpunkteinstellverfahren
Appareil de micro-injection et procédé de mise au point automatique

(30) Priority: 29.06.2006 JP 2006179705
(43) Date of publication of application: 02.01.2008
(73) Proprietor: FUJITSU LIMITED, Kawasaki-shi, Kanagawa 211-8588 (JP)
(72) Inventor: Ando, Moritoshi, Nakahara,Kawasaki-shi Kanagawa 211-8588 (JP); Youoku, Sachihiro, Nakahara-Kawasaki-shi Kanagawa 211-8588 (JP)
(74) Representative: Haseltine Lake Kempner LLP

(56) References cited:
- EP-A- 1 479 759
- EP-A- 1 595 941
- EP-A1- 1 621 912
- DE-A1- 19 721 084
- US-A- 4 631 581
- US-A1- 2005 163 359
- CZYMMEK ET AL: "Confocal microscopy in mycological research" EXPERIMENTAL MYCOLOGY, ACADEMIC PRESS, LONDON, GB, vol. 18, no. 4, December 1994 (1994-12), pages 275-293, XP022112071 ISSN: 0147-5975

## Description

The present invention relates to methods and devices for the manipulation or processing of microscopically small biological objects, in particular for carrying out the microinjection or microsuction technique on biological cells.

The technique of microinjecting substances into the interior of biological cells with the aid of injection capillaries, which are controlled by a micromanipulator, is of increasing importance in biomedical research, for example in the microinjection of DNA into oocytes, for the creation of transgenic animals, for the fertilisation of human egg cells, for studying the transport power of kidney cells or for studying heart diseases or tumours. A fundamental problem in microinjection with commercially available apparatus relates to the targeting reliability of the movement of the injection capillary. It has previously been necessary to estimate the spatial extent of the cell, in order to be able to find particular cell compartments expediently. To date, therefore, it is impossible to treat sizeable numbers of cells, which have for example been seeded in a differing extent on a carrier, with a reproducible automatic procedure.

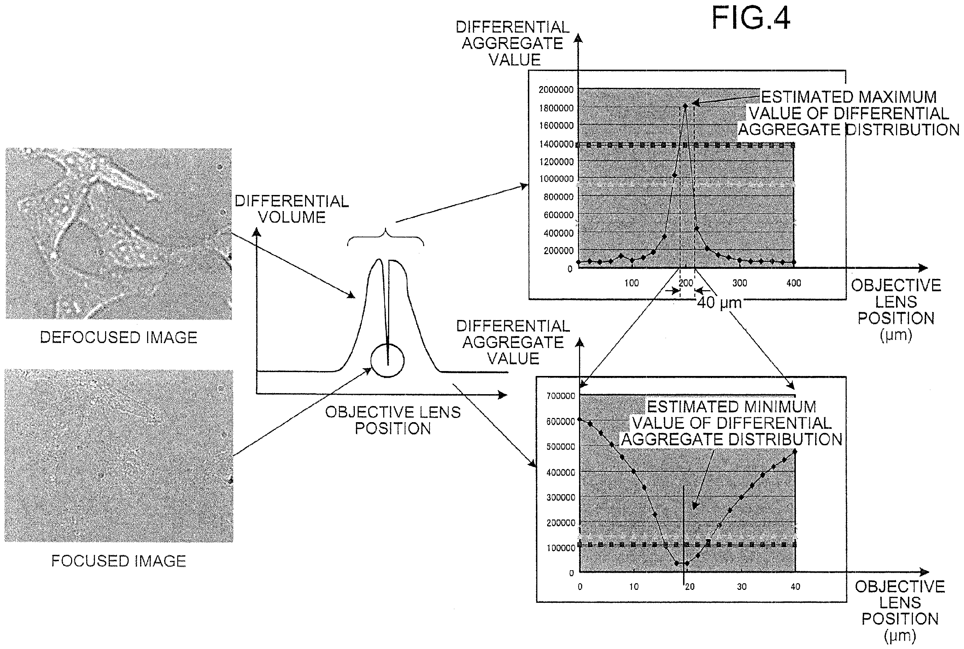

The basic structure of a conventional microinjection system is represented in **Fig.** 4. The microinjection system **40** comprises an injection arrangement **41,** a specimen arrangement **42** and an observation microscope **43.** The injection arrangement **41** consists of an injection capillary **411,** which is connected via a supply line **412** to a supply control and a reservoir (not shown) and can be actuated by a micromanipulator **413.** The specimen arrangement **42** is formed by an X-Y cross stage **421,** on which a cell culture carrier **422** is arranged.

**Fig.** 5 represents a corresponding side view of parts of an injection system, as is known from DE-PS 38 08 531. The biological object **50** contains a compartment **501,** into which the injection is intended to be carried out with the injection capillary **511.** The object **50** is placed on the specimen carrier **522** in the field of view of the observation microscope **53.** In a first step, the injection capillary **511** is arranged above the object **50** by the micromanipulator **513** of the injection arrangement **51,** so that the tip **510** of the injection capillary lies above the compartment **501** in the microscopic image. This preadjustment, which is carried out with a height z₁ above the specimen carrier **522** and at a distance from the front side of the object **50,** is used to record the X-Y coordinates of the compartment **501.** For the injection, the injection capillary **511** is displaced so that an axial (i.e. taking place in the direction of the capillary's longitudinal extent) insertion movement takes place as far as the height z₀ above the specimen carrier **522.** With this axial insertion movement as far as the stored X-Y coordinates of the compartment **501,** damage to the object **50** is minimised. The injection is subsequently carried out by supplying a substance through the supply line **512** and the injection capillary **511.**

The above-described microinjection system known from DE-PS 38 08 531 has the following disadvantages. The system allows only semiautomatic operations. The X-Y coordinates of the compartment **501** (for example cell nucleus) must be stored after microscopic observation. The height z₀, on the other hand, is not available to direct observation and must be preselected beforehand from empirical values. The applicability of the conventional microscope system is therefore restricted to those biological objects whose geometrical structure and whose dimensions are known. Observation of the injection result on the microscopic image is furthermore restricted, so that changes which propagate in a direction perpendicular to the specimen carrier are scarcely perceptible.

Another microinjection system with an image processing technique is known from EP-B-0 292 899. In this system, which has essentially the same structure as the system according to **Fig.** 4, a monitor display of the microscopic image of the object to be treated is provided, on which marking can be carried out with computer control on a selected compartment and its coordinates in the X-Y plane of the specimen carrier can be received automatically by a control computer. This allows the above-described preadjustment to be replaced by marking on the monitor. Nevertheless, the aforementioned disadvantages are not overcome even with this microinjection system. In order to be able to position the tip of the injection capillary as accurately as possible, it is necessary to carry out test injections in the course of which Z coordinates of system different from a system of observing the cell, but this method had a problem that a microinjection apparatus must have at least two lines of optical system, making a configuration of the apparatus complicated, or making the apparatus expensive or reducing the usability and maintainability of the apparatus.

The method of observing the cell conventionally was a differential interference method and a phase difference contrast optical system, but these methods, developed for making the cell more easily viewable in visual observation, had a problem that an edge of the cell is overemphasized, and therefore, were not suitable for an automation of the cell focal point adjustment.

DE 197 21 084 A1 discloses a method and apparatus for processing biological cells through microinjection. In particular the method disclosed includes taking images of a cell in a Petri dish at a plurality of focal planes through out the cell until a three-dimensional representation of the cell can be determined. From this information the injection of material into the cell can be controlled, including making automatic real-time adjustments to the microinjection process.

EP 1479759 A2 discloses an apparatus for injecting a substance into a cell, the apparatus comprising a cell input, cell output, flow path between the input and output and a trapping part arranged in the flow path, wherein an inserting part is used to facilitate the injection of a substance into a cell trapped in the trapping part using a needle.

US 2005/0163359 A1 discloses a method for supporting cell image analysis, wherein a fluorescent image is used to specify a position of a cell, and a bright-field image or a phase difference image is used to specify a cell region of a cell.

### SUMMARY OF THE INVENTION

It is an object of the present invention to at least partially solve the problems in the conventional technology.

Aspects of embodiments of the invention provide a microinjection apparatus and a method of automatically adjusting a focal point of a lens relative to a cell for injecting an object into the cell with a needle, both as defined in the claims. The claims define the scope of the invention.

The above and other objects, features, advantages and technical and industrial significance of this invention will be better understood by reading the following detailed description of presently preferred embodiments of the invention, when considered in connection with the accompanying drawings.

### BRIEF DESCRIPTION OF THE DRAWINGS

Fig. 1 is an explanatory diagram for description of the microinjection into an adherent cell;
Fig. 2A is an explanatory diagram for description of features of an automatic focal point adjustment method according to the first embodiment;
Fig. 2B is an explanatory diagram for description of features of an automatic focal point adjustment method according to the first embodiment;
Fig. 2C is an explanatory diagram for description of features of an automatic focal point adjustment method according to the first embodiment;
Fig. 3 is an explanatory diagram for schematic description of a cell state measurement in the automatic focal point adjustment method according to the first embodiment;
Fig. 4 is an explanatory diagram for schematic description of a cell focal position measurement in the automatic focal point

It is an object of the invention to provide improved methods and devices for the treatment of biological objects, which have an increased spatial accuracy, an extended application range and in particular the possibility of automation.

This object is achieved by the subject-matter of Claims 1 and 10, respectively. Advantageous embodiments of the invention may be found in the dependent claims.

A first essential aspect of the invention consists in changing from conventional microinjection with two-dimensional image processing to treatment of microscopic biological objects (for example cells, cell groups or cell compartments) by using three-dimensional image processing. After per se known deconvolution microscopic imaging of the spatial shape of the object to be studied, the image processing comprises acquisition of the spatial coordinates of predetermined positions of an object region, which for example delimit a cell compartment from the surroundings, and actuation of the processing means as a function of the three spatial coordinates. In contrast to the known microphotometric obtaining of internal relative coordinates, the spatial coordinates relate absolutely to an external surrounding reference system (processing system) in which the processing means are moved.

The treatment to be carried out may be microinjection, suction, mechanical processing and/or application of electrical potentials. The processing means may accordingly be an injection tip, a mechanical processing means or an electrode, which respectively have dimensions that are adapted to the size of the object region to be treated. The processing means are actuated by a computer-controlled manipulator as a function of the acquired spatial coordinates of the object region to be treated and the current spatial coordinates of the processing means.

The treatment system according to the invention has the essential advantage that elaborate preadjustments or test injections become superfluous. This saves on processing outlay and time and, if the vitality of the biological objects has a time limitation, it may also increase the success ratio of the treatment. The object treatment is furthermore fully automatable. Destruction of the injection capillary by touching the specimen carrier can be avoided.

According to another important aspect of the invention, three-dimensional image processing is carried out not only to obtain target coordinates of the object treatment, but also in real-time operation during the processing in order to influence or monitor its progress. To this end the manipulator control employs the temporal evolution of the acquired spatial coordinates, or according to a preferred embodiment the change in the coordinates (difference coordinates). This makes it possible to form an automatic control loop with which the duration, the pressure and/or the volume of an injection or the suction or another kind of activation of the processing means is regulated.

Further advantages and details of the invention will be described below with reference to the appended drawings, in which:

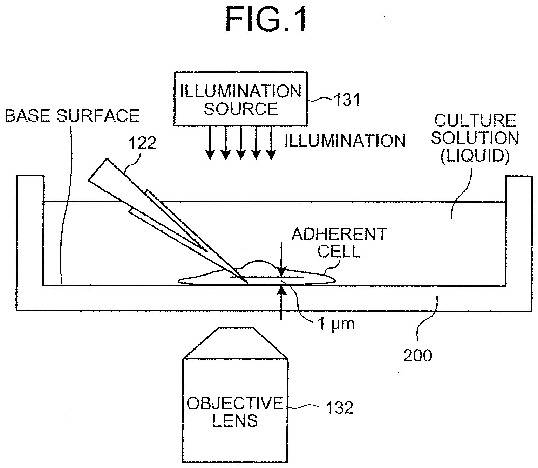

**Fig.** 1 shows a schematic representation of objects to be studied, in order to illustrate the coordinate acquisition;

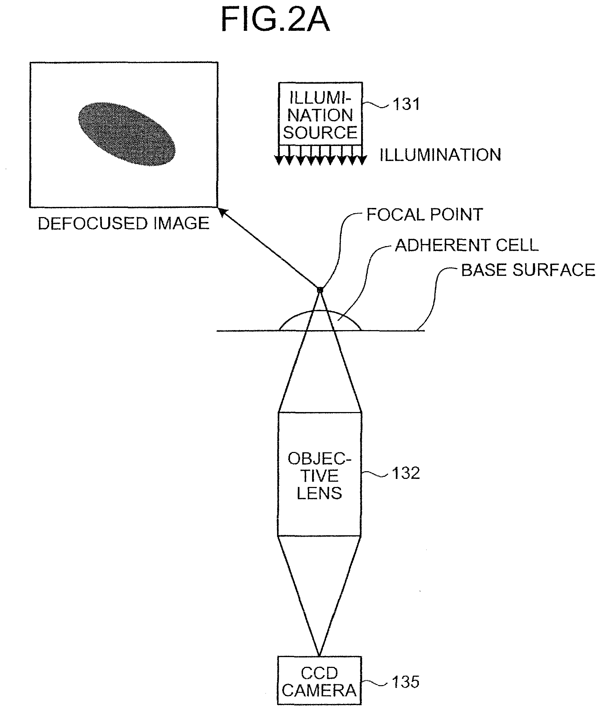

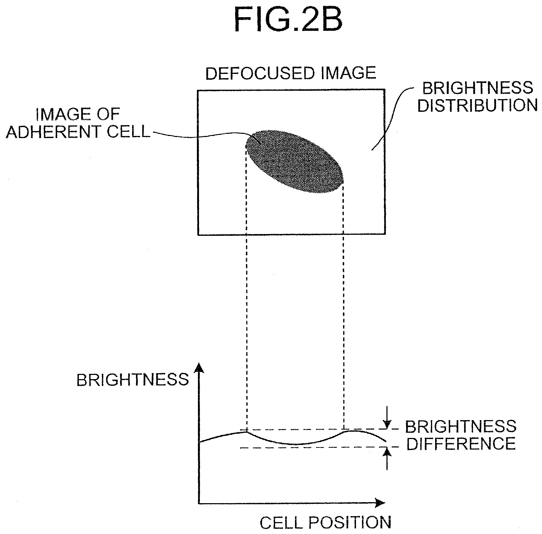

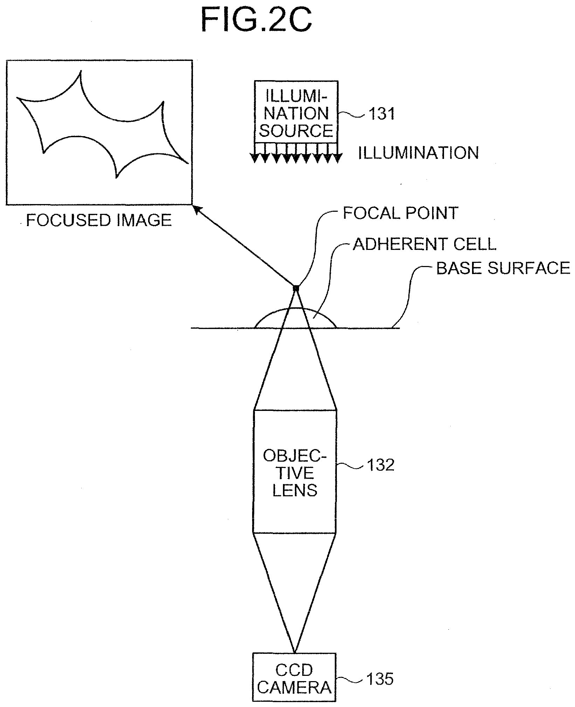

**Fig.** 2 shows a flow chart to explain the procedure according to the invention;

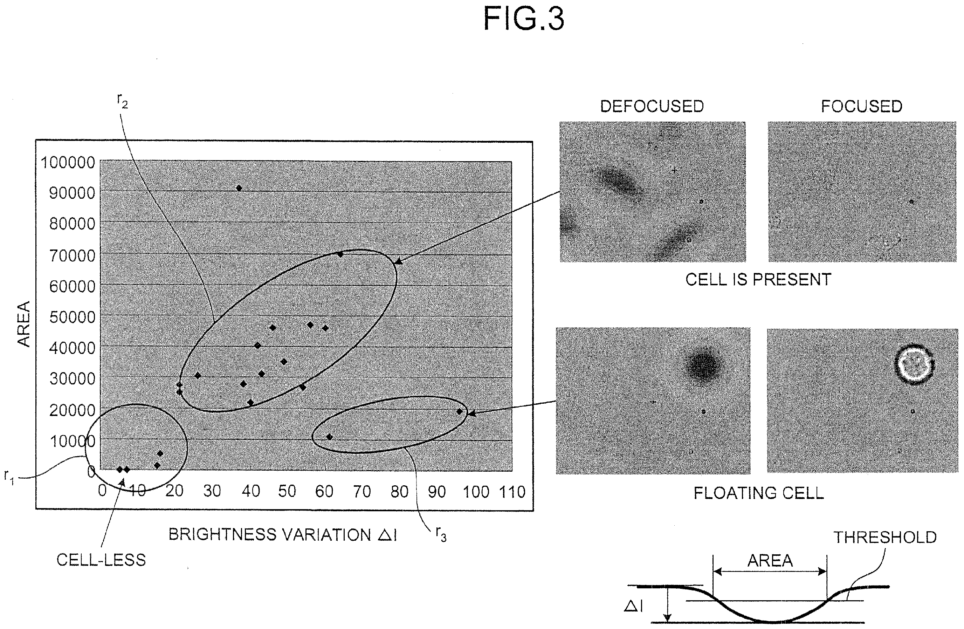

**Fig.** 3 shows a schematic plan view of a specimen carrier;
**Fig.** 4 shows a perspective view of a conventional microinjection system (prior art);

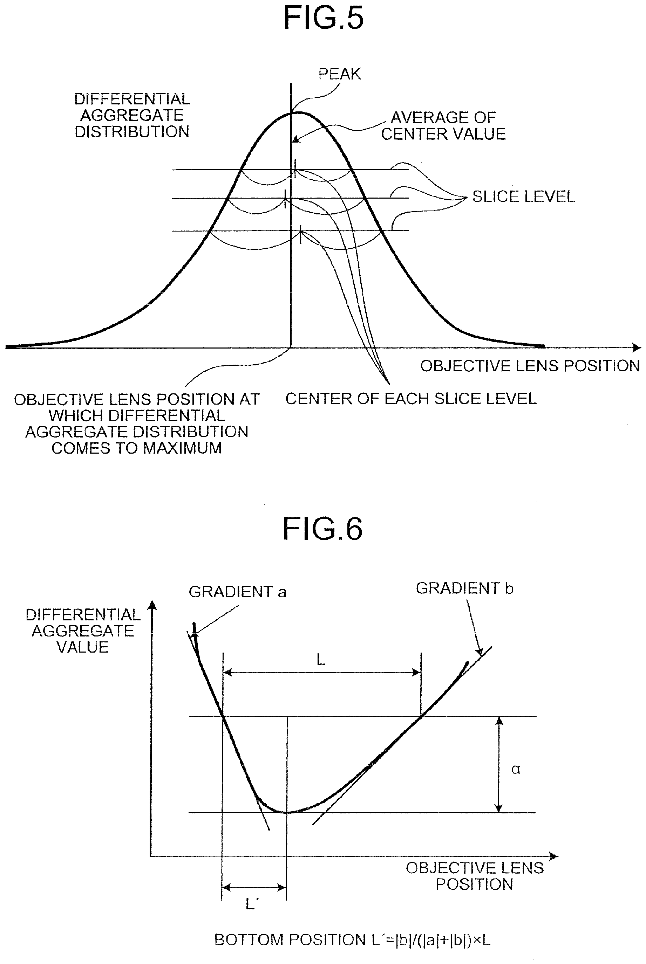

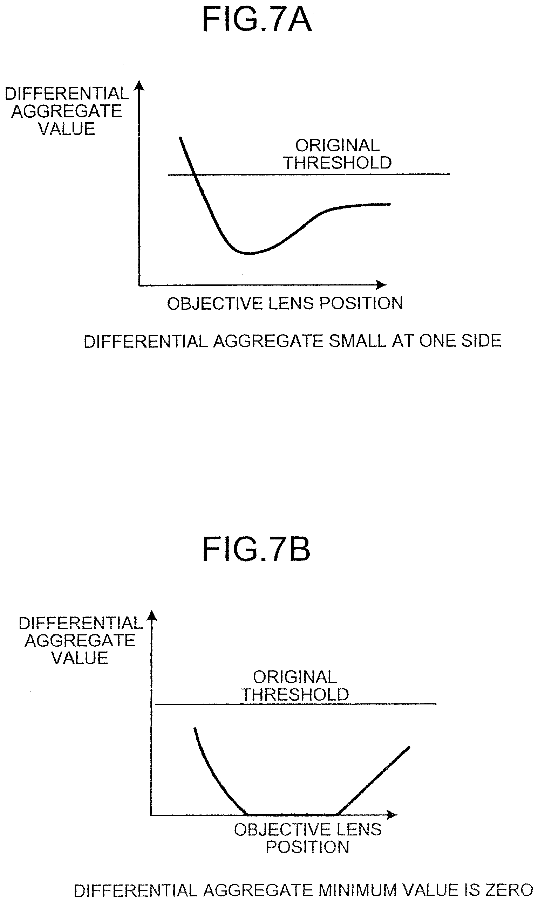

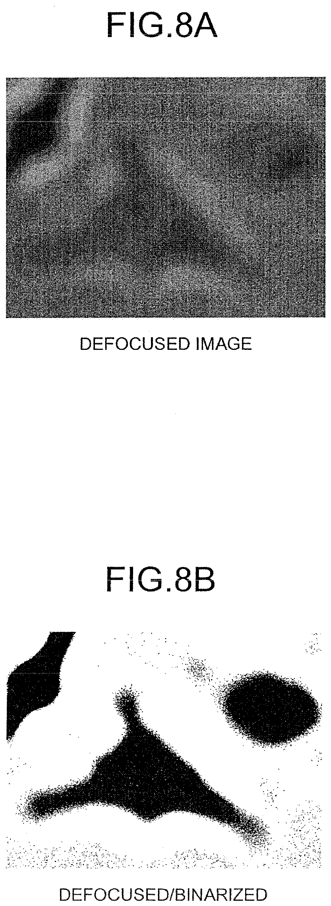

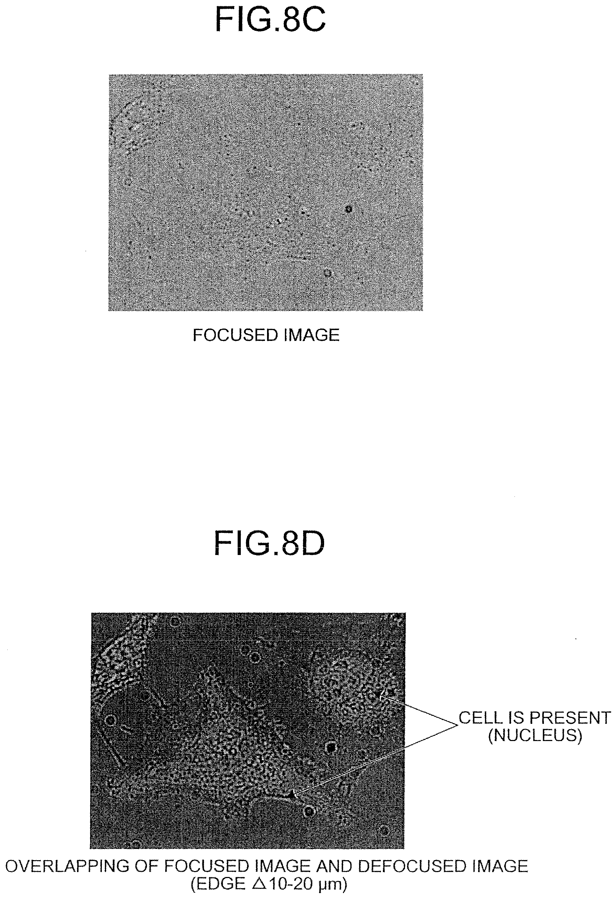

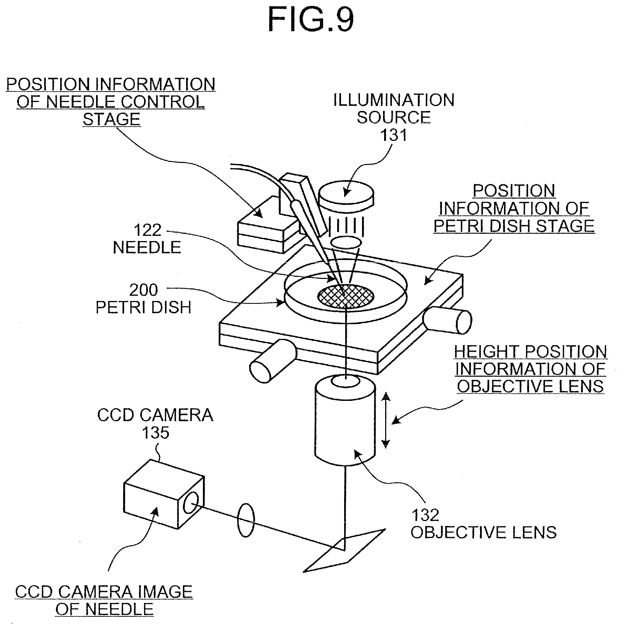

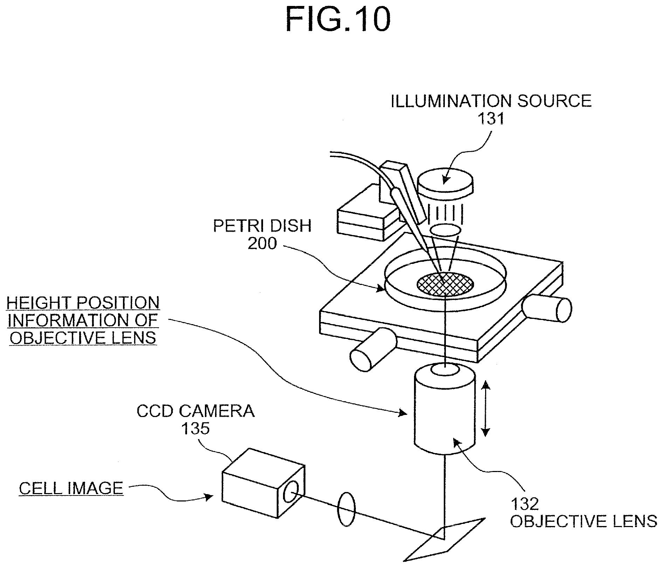

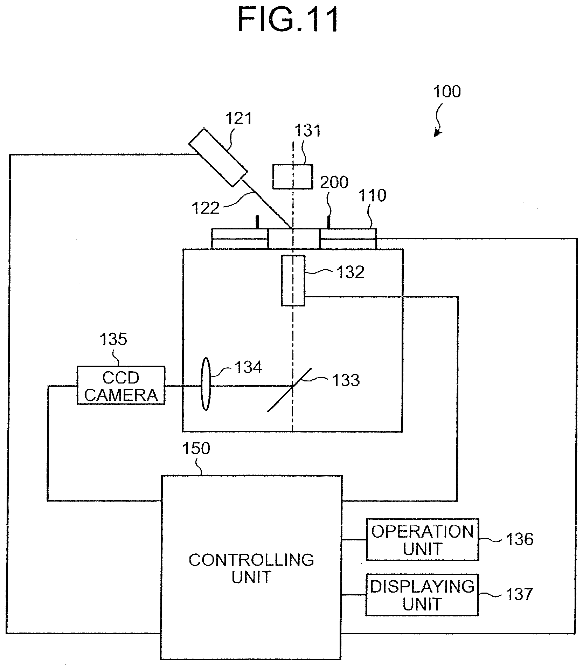

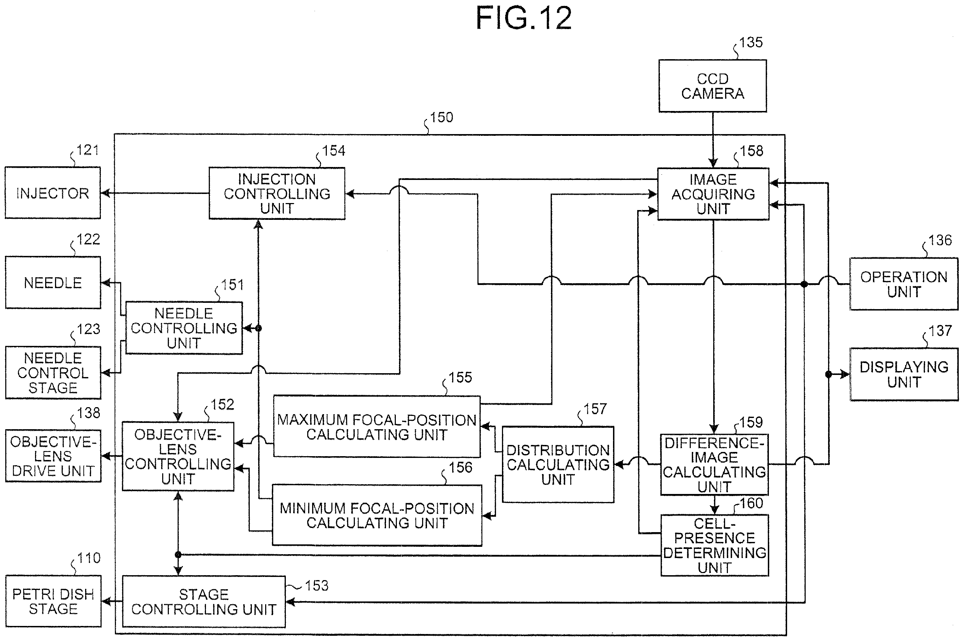

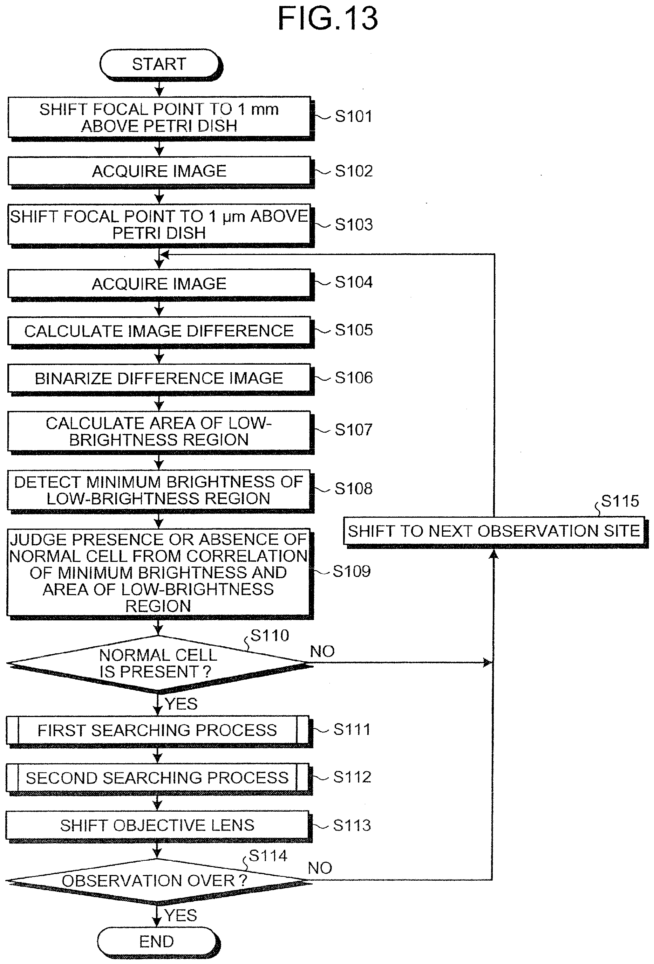

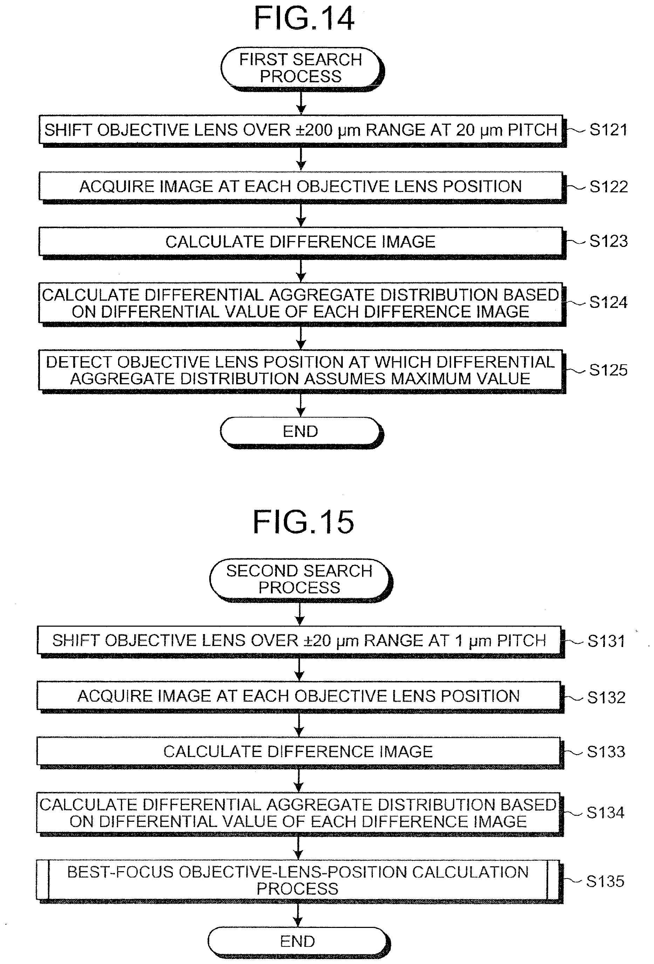

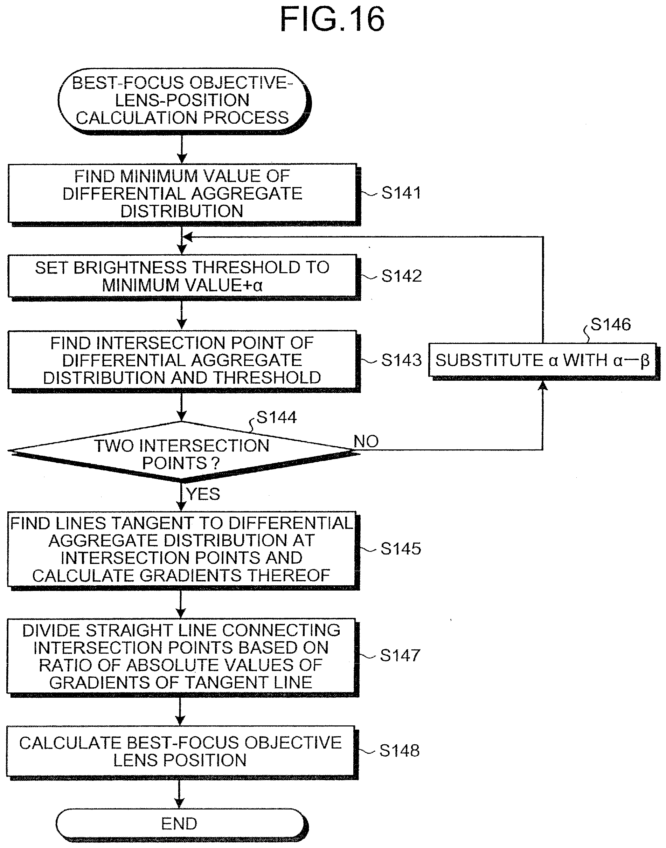

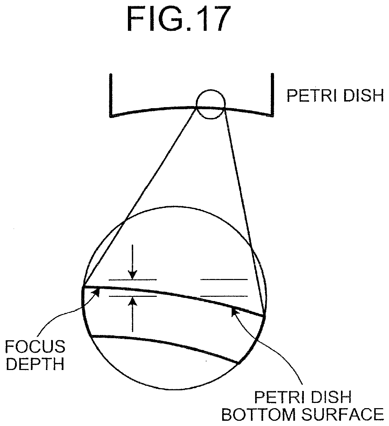

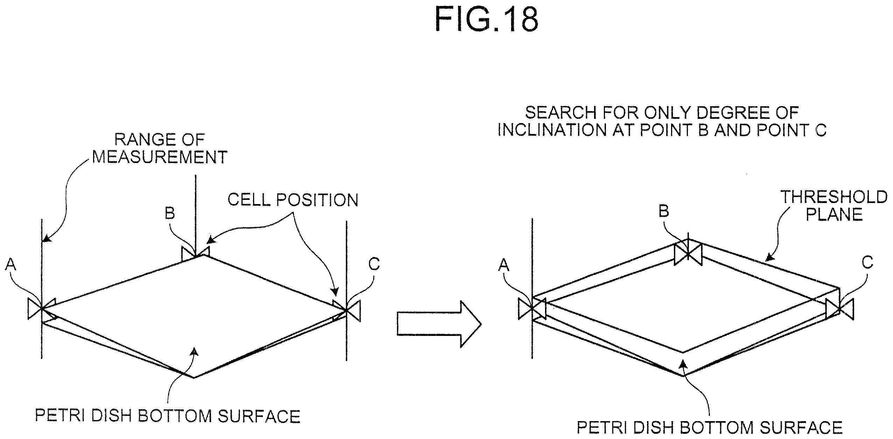

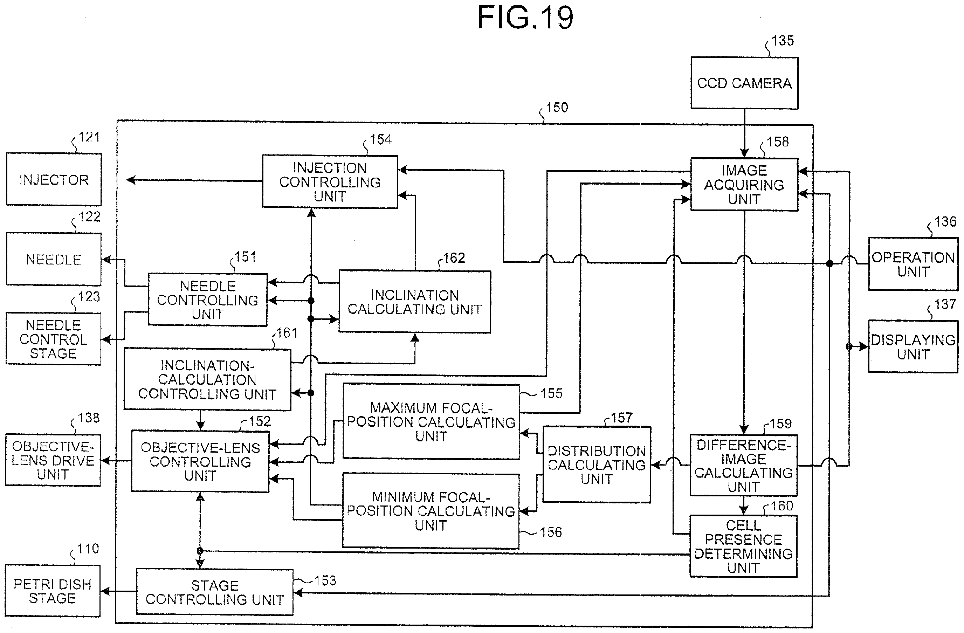

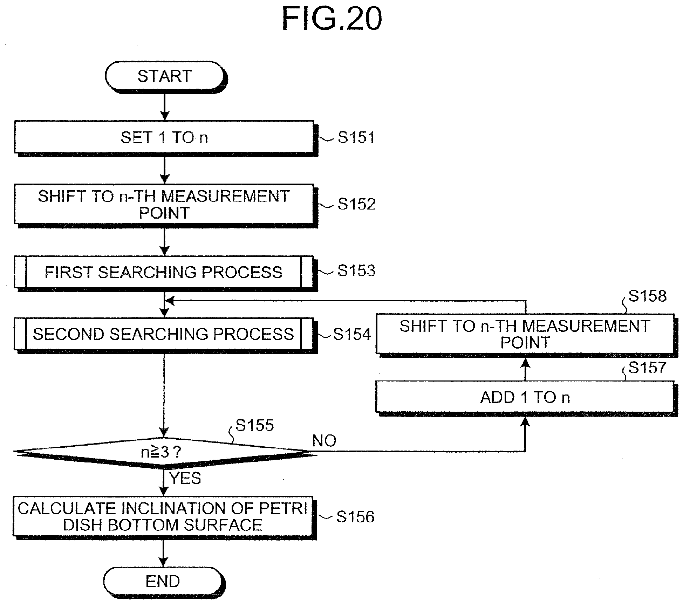

**Fig.** 5 shows a schematic side view of a conventional microinjection system (prior art).

Details of the invention will be described below with reference to the microinjection of biological cells. The invention is not however restricted to microinjection, rather it is also applicable in other treatment techniques.

**Fig.** 1 shows a schematic perspective view of three cells **10A, 10B** and **10C,** respectively with cell compartments **101A, 101B** and **101C.**

The cells to be treated are placed in optically transparent gel embedding. As an alternative, however, it is also possible for the cells to rest on an essentially flat specimen support. The optical axis of the observation microscope extends parallel to the Z axis. The bearing surface of the specimen extends essentially in the X-Y direction. The specified coordinate system lies in a reference system fixed with respect to the specimen support. According to the invention, a polar coordinate system may for example also be used instead of the Cartesian coordinate system.

The image acquisition is carried out with microscopic imaging equipment. This may be a conventional light microscope or a laser scanning microscope. A multiplicity of optical sections of the objects to be studied are recorded, according to different focal planes (focus heights above the specimen support) as is known from the conventional three-dimensional reconstruction techniques. Since the optical sections represent convolutions of the real image with the characteristic imaging properties of the optical system, deconvolution with an equipment-specific transfer function is necessary. This deconvolution may relate to the entire field of view of the microscope or a subregion. For example, the deconvolution may be restricted to a cube enclosing the objects to be treated, which has for example dimensions of 130·130·40 µm in the form schematically represented in **Fig.** 1. In this region, for example, about 80 optical sections may have been recorded (respectively in different focal planes) in order to represent the three represented objects in a real quasi-spatial or perspective fashion after deconvolution with the transfer function.

After imaging of the cell spatial shape, processing of the topology and morphology of the cell volume are carried out. Predetermined regions to be treated are selected, either by an operator marking them on a display means or by the control system recognizing them automatically based on characteristic image properties. Such characteristic image properties may, for example, be fluorescent signals of position-selectively arranged marking substances or characteristic geometrical patterns. The coordinates of the cell regions to be treated, relative to the specimen support i.e. in the coordinate system X, Y, Z, are received by the control system of the microinjection instrument after they have been recognized (marking or recognition of image properties). Since the coordinates of the specimen support relative to the micromanipulator, and of the micromanipulator relative to the tip of the injection capillary, are known, the actuation of the injection capillary may therefore be carried out essentially at the same time as the imaging and coordinate acquisition.

Besides the coordinate acquisition, the processing of the topology and morphology may furthermore involve a volume calculation (cell or cell components). As a result, the volume of the substance to be injected is predetermined and the injection arrangement is driven accordingly.

An inventive embodiment of a microinjection system will be explained below with reference to the flow chart in **Fig.** 2. After a calibrating measurement (see below) the method sequence initially contains two procedures, which relate respectively to the acquisition and analysis of cell data (method branch **21**) and to the acquisition and analysis of system data (method branch **22**).

The processing of the method branches **21** and **22** may be carried out in parallel or alternately; if the system settings are constant, the system data may need to be acquired only once. Method branch **21** initially contains first imaging of the cell to be treated, or at least one region of the cell. After digital data acquisition, data analysis is carried out for quantitative recording of the cell geometry. This includes recording the topology and morphology and optionally determining measures to improve the imaging quality (contrast enhancement or the like). Lastly, the spatial coordinates in the fixed reference system (processing system) are determined and a micromanipulator is controlled as a function of the acquired spatial coordinates in order to actuate the injection capillary and supply the substance to be injected (operation **23**). The procedure in method branch **22** is carried out similarly. The data analysis in this case involves in particular an image processing step to recognize characteristic parts of for example the capillary tip.

The flow chart according to **Fig.** 2 furthermore shows a method branch **24,** which illustrates the use of the system according to the invention as a control loop.

Specifically it is possible to carry out new imaging of the regions to be treated, with coordinate acquisition, simultaneously or alternately with small intervals (for supplying the substance to be injected). The current coordinates of the cell region being treated are compared with target coordinates. For example the volume of a cell compartment, into which a substance is injected, is compared with a target volume. So long as the target volume has not been reached, the substance continues to be injected via the manipulator or the supply controller. Otherwise, the injection is ended. Instead of monitoring the volume, it is also possible to monitor for example the injection duration, the injection pressure, the residence time of the capillary tip in the cell, the penetration depth of the capillary, the position of the microinjection and/or the path of the microinjection.

The inventive embodiment of an object processing as a control loop for the first time offers the opportunity to verify the success of the object processing. Success verification is of great importance particularly for microinjection systems. In conventional systems, the results of the microinjection can only be observed by inflating the cell being treated. This however implies a morphology change, as a result of which the cell will in general already have suffered irreversible damage. This disadvantage can be avoided by monitoring the progress and the result of the microinjection in real time or quasi-real time simultaneously with the injection, by observing a spatial shape image of the cell region being treated.

Another essential advantage of the inventive simultaneous cell processing and spatial image processing relates to improved verification of the processing system. Malfunctions of the processing means can be found and corrected in real time. For instance, the determination of new cell sizes in method branch **24** according to **Fig.** 2 may in the broadest sense also comprise finding a malfunction and automatically or manually replacing the malfunctioning part. In the case of microinjection systems, for example, the injection capillary may become clogged. This is the case in particular when injecting highly viscous suspensions containing protein. As soon as the cell region being treated shows an unchanged image after the three-dimensional image processing when the injection pressure is applied to the injection capillary, then reduced permeability (or impermeability) of the capillary is established and the capillary is changed by a capillary changer.

The simultaneous acquisition of object and system data for the first time allows accurately positioned injection into multicellular cultures. In multicellular cultures, the biological cells are arranged not as a monolayer on the specimen carrier (culture carrier) but as a multilayered irregular clump, for example of agglomerates or spheroids. One example of a multicellular culture is biopsy material in which a substance is injected at predetermined positions and, for example, diffusion of the substance through the cell matrix is intended to be observed. In this case, not just one cell but a whole group of cells will be imaged and processed for the coordinate acquisition. The system according to the invention makes it possible not only for a particular position to be selected in such a cell group, and found in an accurately positioned way, but also for the injection direction to be selected in a predetermined fashion. In general, the selection of a preferred injection direction by changing the alignment of the specimen carrier relative to the injection system is also possible for the processing (in particular injection) of individual cells.

The success verification therefore also comprises the possibility of observing the capillary movement during the introduction and triggering the application of pressure as soon as a target position is reached.

The control loop according to method branch **24** in **Fig.** 2 is automatable. If a fluorescent material is injected, for example, then recording a fluorescent signal (time dependency of the fluorescent intensity) allows information to be obtained about the quantitative result of the injection.

With the inventive embodiment of a control loop, acquisition and processing of the coordinates of the treatment means (for example an injection tip) may thus also be provided simultaneously with the acquisition of the coordinates of the cell region. To this end, the treatment means is provided with a specific identifier (image pattern or fluorescent markers).

The calibrating measurement is used to record the transfer function of the optical system. It therefore only needs to be carried out once for a particular microscope setting, or for particular optical specimen properties. It is not absolutely necessary to carry out a calibrating measurement. If the imaging system has defined, known properties (for example manufacturer data for the optical components) then the transfer function may also be determined by a calibrating calculation instead of a calibrating measurement. In order to record the transfer function, a specimen support having a test object with a known pattern or pattern geometries is subjected to image acquisition with a multiplicity of focal planes, which is the same as that to which the objects to be treated will later be subjected. The test object may for example be formed by gold particles (diameter for example about 60 mm) or fluorescent plastic particles (diameter for example about 200 nm), which rest on the specimen support or are distributed like the cells in a gel. The observed intensity distribution in the respective focal plane can be converted into the transfer function by using the theoretically known real image.

**Fig.** 3 shows an example of a specimen or culture carrier **321** according to the invention, which comprises a specimen region **322** and a calibrating region **323.** The specimens **30** to be treated are arranged in the specimen region **322.** In the calibrating region **323,** there are arrangements of test objects **31** such as those which have been mentioned by way of example above. Such a specimen carrier readily makes it possible to briefly interrupt the object processing with little outlay, and to carry out a calibrating measurement for test purposes or for recalibration when system settings have changed.

According to a preferred embodiment both the calibrating measurement and the imaging of the cell region to be treated, during the microinjection, may be carried out with a continuously moved objective lens (in a conventional microscope) or with a continuous focal plane (in a scanning microscope). The scanning of the focal planes through the object region of interest may, for example, take place with about 80 image acquisitions per second. With such a high image acquisition rate the case of injections, in the shockwave at the start of injection (when changing from holding pressure to injection pressure) and its effect (forming striations) may be observed in real time. This is not possible with conventional systems. This procedure furthermore has the advantage that vibrations, which occur in the event of discrete focus adjustments, can be fully avoided without thereby entailing a problem for the three-dimensional volume reconstruction.

A device according to the invention consists essentially of a processing arrangement having a micromanipulator and processing means, a specimen arrangement for example in the form of an X-Y cross stage with a specimen support, an observation microscope and a control unit. In the case of an automated processing system (particularly a microinjection system), it is possible to construct the processing arrangement without an observation microscope. In this case it is possible merely to provide an optical system for the image acquisition, which is adapted to the requirements of rapid scanning of the focal planes and recording optics which are as simple as possible. If however an observation microscope is used, then this may be a light microscope or a confocal microscope. The invention may in particular be combined with the technique of confocal real-time microscopy, as is described for example by R. Juskaitis et al. in "Nature" (volume 383, p. 804, 1996). The control unit contains imaging means for representing the spatial shape of the object to be studied and means for coordinate acquisition of at least one region to be treated, of the object. The imaging means may comprise a perspective display or defined imaging of the image features in a memory, which is interrogated for particular image features during the coordinate acquisition.

A microinjection system according to the invention is readily automatable. If for multiple injection, for example, a multiplicity of biological objects are treated on a specimen carrier, subsequently stored in an incubator and then treated again, then it is simple to apply markings on the specimen carrier in order to define a coordinate system and find the objects again. The applicability as a microinjection system is significantly extended. Thus, the cells to be treated do not need to have their geometrical properties known in advance. A decision as a function of volume properties of the cell may also be integrated into the injection process. For example, if a marked cell compartment **101C** (see **Fig.** 1) has a volume less than a particular threshold value, then the injection may be prevented for this cell compartment or the injection may be restricted merely to this cell compartment. Owing to the independence from empirical values, the accuracy and reproducibility of the system are significantly increased.

## Claims

1. A microinjection apparatus (100) for injecting an object into a cell with a needle (122), the microinjection apparatus comprising:
an image acquiring unit (158) configured to:
acquire a first image, that is an image of the cell, at a first focal position;
set a standard brightness using the brightness of the first image; and
acquire a second image, that is an image of the cell, at a second focal position, and
a state deciding unit (160) configured to decide a state of the cell by evaluating a level of brightness in a difference image relative to the standard brightness, and an area of a region in the difference image having a brightness lower than a predetermined threshold,
the state deciding unit (160) being further configured to obtain the difference image from the first image and the second image.

2. The microinjection apparatus (100) according to claim 1, wherein the state deciding unit (160) is configured to decide the state of the cell by evaluating a correlation between the level of brightness and the area of the region in the difference image.

3. The microinjection apparatus (100) according to claim 1, wherein the state of the cell includes at least one of presence or absence of the cell, life or death state of the cell, and adherent state of the cell.

4. The microinjection apparatus (100) according to claim 1, further comprising:
a first focal position detecting unit (155) configured to detect a first focal position of the image acquiring unit at which a differential aggregate distribution according to focal positions of a first focal point interval assumes a maximum value as calculated based on a difference image of the reference image and of the images of the cell acquired by the image acquiring unit (158) at focal positions of the first focal point interval of the image acquiring unit (158), at an observation position on the base surface at which the state of the cell is measured by the state deciding unit (160); and
a second focal position detecting unit (156) configured to detect a second focal position of the image acquiring unit (158) at which a differential aggregate distribution according to focal positions of a second focal point interval assumes a minimum value as calculated based on a difference image of the reference image and of the images of the cell acquired by the image acquiring unit (158) at focal positions of the second focal point interval narrower than the first focal point interval within a predetermined range including the first focal position detected by the first focal position detecting unit (155) at the observation position.

5. The microinjection apparatus (100) according to claim 4, further comprising an inclination calculating unit (162) configured to calculate an inclination of the base surface based on the second focal position and the first focal position at least at three observation positions on the base surface.

6. The microinjection apparatus (100) according to claim 5, further comprising a distance keeping unit (151) arranged to keep a constant distance between the base surface and a leading edge of the needle (122) irrespective of the observation positions, according to calculated inclination of the base surface.

7. A method of automatically adjusting a focal point of a lens relative to a cell for injecting an object into the cell with a needle (122), the method comprising:
acquiring with an image acquiring unit (158) a first image, that is an image of the cell, at a first focal position;
setting a standard brightness using the brightness of the first image;
acquiring with the image acquiring unit (158) a second image, that is an image of the cell, at a second focal position; and
deciding a state of the cell by evaluating a level of brightness relative to the standard brightness in a difference image, and an area of a region in the difference image having a brightness lower than a predetermined threshold,
wherein the difference image is obtained from the first image and the second image.

8. The method according to claim 7, wherein the deciding includes deciding the state of the cell by evaluating a correlation between the level of brightness and the area of the region in the difference image.

9. The method according to claim 7, wherein the state of the cell includes at least one of presence or absence of the cell, life or death state of the cell, and adherent state of the cell.

10. The method according to claim 7, further comprising:
first detecting including detecting a first focal position of the image acquiring unit (158) at which a differential aggregate distribution according to focal positions of a first focal point interval assumes a maximum value as calculated based on a difference image of the reference image and of the images of the cell acquired by the image acquiring unit (158) at focal positions of the first focal point interval of the image acquiring unit (158), at an observation position on the base surface at which the state of the cell is decided at the deciding; and
second detecting including detecting a second focal position of the image acquiring unit (158) at which a differential aggregate distribution according to focal positions of a second focal point interval assumes a minimum value as calculated based on a difference image of the reference image and of the images of the cell acquired by the image acquiring unit (158) at focal positions of the second focal point interval narrower than the first focal point interval within a predetermined range including the first focal position detected at the first detecting at the observation position.

11. The method according to claim 10, further comprising calculating an inclination of the base surface based on the second focal position and the first focal position at least at three observation positions on the base surface.

12. The method according to claim 11, further comprising keeping a constant distance between the base surface and a leading edge of the needle (122), irrespective of the observation positions, according to calculated inclination of the base surface.

## Patentansprüche

1. Mikroinjektionsvorrichtung (100) zum Injizieren eines Objekts in eine Zelle mit einer Nadel (122),
wobei die Mikroinjektionsvorrichtung umfasst:
eine Bilderfassungseinheit (158), die konfiguriert ist:
um ein erstes Bild, das ein Bild der Zelle ist, an einer ersten Brennpunktposition zu erfassen;
um eine Standardhelligkeit unter Verwendung der Helligkeit des ersten Bildes einzustellen; und
um ein zweites Bild, das ein Bild der Zelle ist, an einer zweiten Brennpunktposition zu erfassen; und
eine Zustandsentscheidungseinheit (160), die konfiguriert ist, um einen Zustand der Zelle durch Auswerten eines Helligkeitspegels in einem Differenzbild relativ zur Standardhelligkeit, und in einem Bereich einer Region in dem Differenzbild, welcher eine Helligkeit aufweist, die niedriger als ein vorbestimmter Schwellenwert ist, zu entscheiden,
wobei die Zustandsentscheidungseinheit (160) ferner konfiguriert ist, um das Differenzbild aus dem ersten Bild und dem zweiten Bild zu erhalten.

2. Mikroinjektionsvorrichtung (100) nach Anspruch 1, wobei die Zustandsentscheidungseinheit (160) konfiguriert ist, um den Zustand der Zelle durch Auswerten einer Korrelation zwischen dem Helligkeitspegel und dem Bereich der Region in dem Differenzbild zu bestimmen.

3. Mikroinjektionsvorrichtung (100) nach Anspruch 1, wobei der Zustand der Zelle mindestens einen von dem Vorhandensein oder der Abwesenheit der Zelle, einem lebenden oder toten Zustand der Zelle und einem adhärenten Zustand der Zelle umfasst.

4. Mikroinjektionsvorrichtung (100) nach Anspruch 1, ferner umfassend:
eine erste Brennpunktpositions-Erfassungseinheit (155), die so konfiguriert ist, dass sie eine erste Brennpunktposition der Bilderfassungseinheit erfasst, bei der eine differentielle Aggregatverteilung gemäß den Brennpunktpositionen eines ersten Brennpunktintervalls einen maximalen Wert annimmt, wie er auf der Grundlage eines Differenzbildes des Referenzbildes und der Bilder der Zelle berechnet wird, die durch die Bilderfassungseinheit (158) an Brennpunktpositionen des ersten Brennpunktintervalls der Bilderfassungseinheit (158) an einer Beobachtungsposition auf der Basisfläche erfasst werden, an welcher der Zustand der Zelle durch die Zustandsbestimmungseinheit (160) gemessen wird; und
eine zweite Brennpunktpositions-Erfassungseinheit (156), die so konfiguriert ist, dass sie eine zweite Brennpunktposition der Bilderfassungseinheit (158) erfasst, bei der eine differentielle Aggregatsverteilung gemäß den Brennpunktpositionen eines zweiten Brennpunktintervalls einen minimalen Wert annimmt, wie er auf der Grundlage eines Differenzbildes des Referenzbildes und der Bilder der Zelle berechnet wird, die durch die Bilderfassungseinheit (158) an Brennpunktpositionen des zweiten Brennpunktintervalls, das schmaler als das erste Brennpunktintervall ist, innerhalb eines vorbestimmten Bereichs erfasst werden, der die erste Brennpunktposition, die von der ersten Brennpunktpositions-Erfassungseinheit (155) an der Beobachtungsposition erfasst wird, umfasst.

5. Mikroinjektionsvorrichtung (100) nach Anspruch 4, die ferner eine Neigungsberechnungseinheit (162) umfasst, die so konfiguriert ist, dass sie eine Neigung der Basisfläche auf der Grundlage der zweiten Brennpunktposition und der ersten Brennpunktposition an mindestens drei Beobachtungspositionen auf der Basisfläche berechnet.

6. Mikroinjektionsvorrichtung (100) nach Anspruch 5, die ferner eine Abstandshalteeinheit (151) umfasst, die so angeordnet ist, dass sie einen konstanten Abstand zwischen der Basisfläche und einer Vorderkante der Nadel (122) unabhängig von den Beobachtungspositionen entsprechend der berechneten Neigung der Basisfläche hält.

7. Verfahren zum automatischen Einstellen eines Brennpunkts einer Linse relativ zu einer Zelle zum Injizieren eines Objekts in die Zelle mit einer Nadel (122), wobei das Verfahren umfasst:
das Erfassen eines ersten Bildes, das ein Bild der Zelle ist, an einer ersten Brennpunktposition mit einer Bilderfassungseinheit (158);
das Einstellen einer Standardhelligkeit unter Verwendung der Helligkeit des ersten Bildes;
das Erfassen eines zweiten Bildes, das ein Bild der Zelle ist, an einer zweiten Brennpunktposition mit der Bilderfassungseinheit (158); und
das Entscheiden eines Zustands der Zelle durch Auswerten eines Helligkeitspegels relativ zu der Standardhelligkeit in einem Differenzbild, und in einem Bereich einer Region in dem Differenzbild, welcher eine Helligkeit aufweist, die niedriger als ein vorbestimmter Schwellenwert ist,
wobei das Differenzbild aus dem ersten Bild und dem zweiten Bild erhalten wird.

8. Verfahren nach Anspruch 7, wobei das Entscheiden das Entscheiden des Zustands der Zelle durch Auswerten einer Korrelation zwischen dem Helligkeitspegel und dem Bereich der Region in dem Differenzbild umfasst.

9. Verfahren nach Anspruch 7, wobei der Zustand der Zelle mindestens einen von dem Vorhandensein oder der Abwesenheit der Zelle, einem lebenden oder toten Zustand der Zelle und einem adhärenten Zustand der Zelle umfasst.

10. Verfahren nach Anspruch 7, ferner umfassend:
das erste Erfassen, welches das Erfassen einer ersten Brennpunktposition der Bilderfassungseinheit (158) umfasst, bei der eine differentielle Aggregatsverteilung gemäß den Brennpunktpositionen eines ersten Brennpunktintervalls einen Maximalwert annimmt, wie er auf der Grundlage eines Differenzbildes des Referenzbildes und der Bilder der Zelle berechnet wird, die durch die Bilderfassungseinheit (158) an Brennpunktpositionen des ersten Brennpunktintervalls der Bilderfassungseinheit (158) an einer Beobachtungsposition auf der Basisfläche erfasst werden, an welcher der Zustand der Zelle bei der Entscheidung entschieden wird; und
das zweite Erfassen, welches das Erfassen einer zweiten Brennpunktposition der Bilderfassungseinheit (158) umfasst, bei der eine differentielle Aggregatsverteilung gemäß den Brennpunktpositionen eines zweiten Brennpunktintervalls einen minimalen Wert annimmt, wie er auf der Grundlage eines Differenzbildes des Referenzbildes und der Bilder der Zelle berechnet wird, die durch die Bilderfassungseinheit (158) an Brennpunktpositionen des zweiten Brennpunktintervalls, das schmaler als das erste Brennpunktintervall ist, innerhalb eines vorbestimmten Bereichs erfasst werden, der die erste Brennpunktposition, die von der ersten Brennpunktpositions-Erfassungseinheit an der Beobachtungsposition erfasst wird, umfasst.

11. Verfahren nach Anspruch 10, das ferner das Berechnen einer Neigung der Basisfläche auf der Grundlage der zweiten Brennpunktposition und der ersten Brennpunktposition an mindestens drei Beobachtungspositionen auf der Basisfläche umfasst.

12. Verfahren nach Anspruch 11, die ferner das Halten eines konstanten Abstands zwischen der Basisfläche und einer Vorderkante der Nadel (122) unabhängig von den Beobachtungspositionen entsprechend der

## Revendications

1. Appareil de micro-injection (100) destiné à injecter un objet dans une cellule au moyen d'une aiguille (122), l'appareil de micro-injection comprenant :
une unité d'acquisition d'images (158) configurée de manière à :
acquérir une première image, à savoir une image de la cellule, à une première position focale ;
définir une luminosité standard en utilisant la luminosité de la première image ;
acquérir une seconde image, à savoir une image de la cellule, à une seconde position focale ; et
une unité de détermination d'état (160) configurée de manière à déterminer un état de la cellule en évaluant un niveau de luminosité dans une image de différence par rapport à la luminosité standard, et une zone d'une région dans l'image de différence présentant une luminosité inférieure à un seuil prédéterminé ;
l'unité de détermination d'état (160) étant en outre configurée de manière à obtenir l'image de différence à partir de la première image et de la seconde image.

2. Appareil de micro-injection (100) selon la revendication 1, dans lequel l'unité de détermination d'état (160) est configurée de manière à déterminer l'état de la cellule, en évaluant une corrélation entre le niveau de luminosité et la zone de la région dans l'image de différence.

3. Appareil de micro-injection (100) selon la revendication 1, dans lequel l'état de la cellule inclut au moins un état parmi un état de présence ou d'absence de la cellule, un état de vie ou de mort de la cellule, et un état d'adhérence de la cellule.

4. Appareil de micro-injection (100) selon la revendication 1, comprenant en outre :
une première unité de détection de position focale (155) configurée de manière à détecter une première position focale de l'unité d'acquisition d'images, à laquelle une répartition globale différentielle selon des positions focales d'un premier intervalle de point focal prend une valeur maximale, telle que calculée sur la base d'une image de différence de l'image de référence et des images de la cellule acquises par l'unité d'acquisition d'images (158), à des positions focales du premier intervalle de point focal de l'unité d'acquisition d'images (158), à une position d'observation sur la surface de base à laquelle l'état de la cellule est mesuré par l'unité de détermination d'état (160) ; et
une seconde unité de détection de position focale (156) configurée de manière à détecter une seconde position focale de l'unité d'acquisition d'images (158), à laquelle une répartition globale différentielle selon des positions focales d'un second intervalle de point focal prend une valeur minimale, telle que calculée sur la base d'une image de différence de l'image de référence et des images de la cellule acquises par l'unité d'acquisition d'images (158), à des positions focales du second intervalle de point focal plus étroites que celles du premier intervalle de point focal dans une plage prédéterminée incluant la première position focale détectée par la première unité de détection de position focale (155) à la position d'observation.

5. Appareil de micro-injection (100) selon la revendication 4, comprenant en outre une unité de calcul d'inclinaison (162) configurée de manière à calculer une inclinaison de la surface de base, sur la base de la seconde position focale et de la première position focale, au moins à trois positions d'observation sur la surface de base.

6. Appareil de micro-injection (100) selon la revendication 5, comprenant en outre une unité de maintien de distance (151) agencée de manière à maintenir une distance constante entre la surface de base et un bord d'attaque de l'aiguille (122), indépendamment des positions d'observation, selon l'inclinaison calculée de la surface de base.

7. Procédé de mise au point automatique d'un point focal d'une lentille par rapport à une cellule pour injecter un objet dans la cellule au moyen d'une aiguille (122), le procédé comprenant les étapes ci-dessous consistant à :
acquérir, par le biais d'une unité d'acquisition d'images (158), une première image, à savoir une image de la cellule, à une première position focale ;
définir une luminosité standard en utilisant la luminosité de la première image ;
acquérir, par le biais de l'unité d'acquisition d'images (158), une seconde image, à savoir une image de la cellule, à une seconde position focale ; et
déterminer un état de la cellule en évaluant un niveau de luminosité par rapport à la luminosité standard dans une image de différence, et une zone d'une région dans l'image de différence présentant une luminosité inférieure à un seuil prédéterminé ;
dans lequel l'image de différence est obtenue à partir de la première image et de la seconde image.

8. Procédé selon la revendication 7, dans lequel l'étape de détermination consiste à déterminer l'état de la cellule en évaluant une corrélation entre le niveau de luminosité et la zone de la région dans l'image de différence.

9. Procédé selon la revendication 7, dans lequel l'état de la cellule inclut au moins un état parmi un état de présence ou d'absence de la cellule, un état de vie ou de mort de la cellule, et un état d'adhérence de la cellule.

10. Procédé selon la revendication 7, comprenant en outre :
une première étape de détection consistant à détecter une première position focale de l'unité d'acquisition d'images (158), à laquelle une répartition globale différentielle selon des positions focales d'un premier intervalle de point focal prend une valeur maximale, telle que calculée sur la base d'une image de différence de l'image de référence et des images de la cellule acquises par l'unité d'acquisition d'images (158), à des positions focales du premier intervalle de point focal de l'unité d'acquisition d'images (158), à une position d'observation sur la surface de base à laquelle l'état de la cellule est déterminé à l'étape de détermination ; et
une seconde étape de détection consistant à détecter une seconde position focale de l'unité d'acquisition d'images (158), à laquelle une répartition globale différentielle selon des positions focales d'un second intervalle de point focal prend une valeur minimale, telle que calculée sur la base d'une image de différence de l'image de référence et des images de la cellule acquises par l'unité d'acquisition d'images (158), à des positions focales du second intervalle de point focal plus étroites que celles du premier intervalle de point focal dans une plage prédéterminée incluant la première position focale détectée à la première étape de détection à la position d'observation.

11. Procédé selon la revendication 10, comprenant en outre l'étape consistant à calculer une inclinaison de la surface de base sur la base de la seconde position focale et de la première position focale, au moins à trois positions d'observation sur la surface de base.

12. Procédé selon la revendication 11, comprenant en outre l'étape consistant à maintenir une distance constante entre la surface de base et un bord d'attaque de l'aiguille (122), indépendamment des positions d'observation, selon l'inclinaison calculée de la surface de base.
